# EUROPEAN PATENT SPECIFICATION

(11) **EP 4 069 152 B1**
(45) Date of publication and mention of the grant of the patent: **03.09.2025**
(21) Application number: 20808215.6
(22) Date of filing: 26.10.2020
(51) Int. Cl.: A61F 2/32, A61F 2/34, A61F 2/36

(54) **ENDOPROSTHESIS**
ENDOPROTHESE
ENDOPROTHÈSE

(30) Priority: 02.12.2019 PL 43204219
(43) Date of publication of application: 12.10.2022
(73) Proprietor: Indywidualna Specjalistyczna Praktyka Lekarska, 60-614 Poznan (PL)
(72) Inventor: ROGALA, Piotr, 60-615 Poznan (PL); ROGALA, Jan, 60-615 Poznan (PL); ROGALA, Stanislaw, 60-615 Poznan (PL); ZAWADZKI, Pawel, 62-060 Witobel/Steszew (PL)
(74) Representative: Urbanska-Luczak, Barbara
(86) International application number: PCT/PL2020/000082
(87) International publication number: WO 2021/112700

(56) References cited:
- EP-A1- 0 013 863
- US-A1- 2008 215 160
- US-A1- 2009 105 772
- US-A1- 2010 312 353

## Description

The subject of the invention is an endoprosthesis for implantation in bone surgery, in particular hip surgery, without the use of surgical cement.

It is known from U.S. patent application No. US 2009/0105772 A1, an implant for anchoring on a bone has a plurality of anchoring pins which extend away from a fastening side of the implant and are provided for anchoring the implant in the bone. In different areas of the fastening side of the implant, the pin arrangement and/or the geometry of the pins are chosen differently. In particular, in different areas of the fastening side of the implant, the volume of the anchoring pins per unit of surface area of the fastening side of the implant is different, preferably in such a way that the geometry and/or arrangement of the pins is chosen according to the proportion of bone tissue in the total tissue of the bone substance, opposite which the area of the fastening side is provided, in such a way that, in areas where there is a relatively higher proportion of bone tissue as a whole, less tissue is displaced by the anchoring pins than in areas where there is a relatively smaller proportion of bone tissue.

The PL174976 patent demonstrates endoprosthesis for implantation in osteoarticular surgery composed of acetabulum and femoral head, which have sharp protrusions on the limiting surface, whereas the limiting surfaces of the acetabulum and femoral head have the form of a spherical cap, and the sharp protrusions with mutually parallel axes have correspondingly the form of polygonal needles of varying length placed symmetrically, with each needle contacting the adjacent edge needle at its base, whereas the outer polygonal needle protrusions placed respectively next to the supporting edge and the supporting surface have the shortest length, and the total area of the polygonal protrusions and central polygonal needle is advantageously seven times larger than the area of the sphere on which they were placed.

A German patent description DE 3443109 Al also demonstrates a solution in which the endoprosthesis is more securely fastened through the use of conical irregularities protruding from the limiting surface of the endoprosthesis and pointed towards the connecting surface of the bone. The conical irregularities are separate elements, which have a conical part and a cylindrical part set in the matrix of the acetabulum and in the matrix of the femoral head. For this purpose holes are made in the flat surfaces of the femoral head and on the annular offsets of the external spherical surface of the acetabulum. These holes weaken both the acetabulum and the femoral head of the endoprosthesis in a lateral cross-section, and simultaneously the conical irregularities set in them are at risk of loosening, which leads to unstable embedding of the endoprosthesis in the bone. In a known solution, DE 3443109 AI the conical irregularities are fully pressed into the spongy bone, which increases the contact area, which facilitates the embedding of the endoprosthesis in the bone material. The flaw of this solution is not using the possible optimum bone-endoprosthesis contact area, due to the fact that the conical irregularities are placed in regular spacing on flat surfaces of the femoral head and on the annular offsets of the external spherical surface of the acetabulum, moreover the flat contact surface by directly contacting the bone at the moment of implantation prevents the later growing in of spongy bone in the form similar to natural, which would ensure good shock absorption of loads on the joint.

The CN 105125324 invention demonstrates an improved metal acetabulum outer cup with rough lamina for artificial hip join. The improved metal acetabulum outer cup is characterized in that the cup is made by casting or forging, and a rough medical metal lamina is formed on a corresponding surface, contacting with bones of a patient, of the acetabulum outer cup by material increase manufacturing.

The AU 2015286971 invention relates to an acetabulum for a hip prosthesis, comprising an inner cavity and a rear outer surface facing the bone. In order to be able to implant the acetabulum without using cement, at least some areas of the outer surface are porous and osseointegrative, and the acetabulum is made exclusively of a ceramic material.

In the US 20130190889 invention an artificial hip joint was presented consisting of multi-layer shell core composite structural components, which includes an artificial acetabular bone and an artificial femoral head, which are mutually matched with each other. The artificial acetabular bone has a multi-layer shell core composite structure and is constituted of a ceramic acetabular bone lining, transitional layers, an acetabular bone shell made of a porous metal or a porous alloy or a porous toughened ceramic. The artificial femoral head has a multi-layer shell core composite structure and is constituted of a ceramic spherical shell layer, a transitional layer and a toughened ceramic inner core. The artificial acetabular bone lining and the artificial femoral head spherical shell layer of the hip joint have high rigidity, anti-corrosion and anti-wear performance. In order to improve adhesion and stability the artificial layer of the acetabulum shell and internal layer of the core off the femoral head of the endoprosthesis have high strength and shock resistance. It is a solution which creates micropores.

In the EP 1308141 invention a hip joint endoprosthesis is presented, provided with a plurality meridional incisions, which define as many segments elastically flexible in the radial direction. The inner surface of the socket is provided with one or more grooves, parallel to the proximal rim, wherein these grooves receive corresponding protrusions of a joint insert. The socket additionally comprises one or more frustoconical portions which form bearing surfaces for corresponding frustoconical portions of the joint insert. The inner surface of the socket bounds a polygonal seat which is adapted to engage with a polygonal portion of the joint insert by means of a form fit.

In the EP2338443 patent presents a fastening shell with a teeth structure arranged in an external side of a semi shell, and a milling tool for forming a shape of teeth flanks. The equator-side oriented flanks of the individual teeth form an angle of less than 90 degrees with a component axis such that barbed hook effect is developed in an equator. A path of the tool is curved for forming teeth gaps and formed such that the teeth of a teeth row are consecutively arranged in the teeth gaps and cutting edges of the teeth from the equator towards a pole do not overlap or have a gap of around 2 mm.

The RU2467724 patent discusses endoprosthesis which comprises a pivotally connected cotyloid prosthesis and prosthesis of a proximal end of femoral bone having a stem for intraosseous introduction, a neck and a spherical head. On an external surface of the head, there is a cylindrical section in the form of a flat. The endoprosthesis has a spheroidal internal cavity of a depth exceeding a half-radius of the head with an input hole less than the head diameter and equal to its diameter within the cylindrical section to lock and key the head in the cotyloid endoprosthesis. The cylindrical section faces a junction of the head and the neck at an angle approximately 33-61° to its axis. The head diameter is 22-38 mm, while the neck diameter is 13-14 mm. The invention may provide higher reliability and durability of endoprosthesis function after implantation.

The invention is defined in claim 1. The subject of the invention is an endoprosthesis for implantation in bone surgery, especially of the hip joint, better adapted to provide long-term cementless fixation.

The essence of the invention is an endoprosthesis for implantation in bone surgery of ball-and-socket joints, in particular hip joint, the endoprosthesis consisting of a moving connection provided by a contact surface of each of an endoprosthesis femoral head component and an endoprosthesis acetabular component, whereas each of the endoprosthesis head component and the endoprosthesis acetabular component has a bone contacting surface having a shape similar to a sphere, on which pins are placed, each of the pins having an axis principally parallel to the lengthwise axis of the endoprosthesis, characterized in that each on the bone contacting surfaces of the endoprosthesis head component and the endoprosthesis acetabular component has at least two cutting blades with an arc-shaped cross-section outline, wherein each of the blades creates a groove for removal of the products of cutting.

It is advantageous, when the sphere is a convex shell, on which pins are located, forming the endoprosthesis acetabulum, or when the sphere is a concave shell, on which pins are located, forming the endoprosthesis femoral head.

It is also advantageous, when the endoprosthesis has on a spherical surface, in a zone of the lengthwise axis, advantageously rounded and porous supporting surface.

It is also advantageous, when the pins are placed on a spherical surface forming the limiting surface.

Moreover it is advantageous when the cutting blades have an angle of application ß within a range of 5-15°, advantageously 10°, angle of attack α 35-45°, advantageously 40°, and a wall thickness H of the cutting blade wall amounting to 0.25-1 mm, advantageously 0.5 mm, and a H1 height of the cutting blade is 2-3 times higher than a H2 height of a opposite wall 4 of the groove 5.

It is in particular it is advantageous when the supporting surface has a D2 diameter with a value of 1/2-1/3 of the D1 diameter of the femoral head.

It is advantageous when the femoral head endoprosthesis has a cylindrical elongation with an A angle in relation to a centre point within a range of 30 to 50 degrees and when the end of the grooves is situated in a zone of technological gripping devices, and the distance "z" between the bases of adjacent pins is at least 100 micrometers.

It is also advantageous when the pins have a shape of a cone.

It is also advantageous when the pins have shaped side surfaces.

And it is also advantageous when the height of the pins on a surface of the endoprosthesis acetabulum shell decreases from an edge to the supporting surface, the height of the pins on a surface of the convex endoprosthesis femoral head shell decreases from the lengthwise axis of the endoprosthesis to an external direction.

It is advantageous when an insert is introduced on not on an internal spherical surface of the endoprosthesis acetabulum.

The use of the solution presented in the invention is expected to provide the following technical and utility effects:
- maximum reduction of micro-movements and loosening of the acetabulum and cap, due to the density of pins with a maximally increased adhesion surface in the bone-implant relationship,
- elimination of the use of glues/cements, which after some time cause stem endoprostheses to loosen,
- appropriate distribution of forces between the bone and the endoprosthesis, as well as a much less invasive implantation procedure and the possibility of using in patients within a larger age range,
- much more advantageous growth of side (adhesive) surface due to the use of a multipin area,
- longer period of use of the endoprosthesis,
- relatively easy replacement in case repeated implantation is required,
- bringing the shape of the endoprosthesis closer to biological model,
- endoprosthesis may be implanted in patients with advanced osteoporosis without the use of surgical cement (glue),
- minimised implantation procedure minimises the degree of trauma to bone tissue,
- the design of the endoprosthesis cap protects blood vessel which supply blood to the proximal end of the femoral bone.

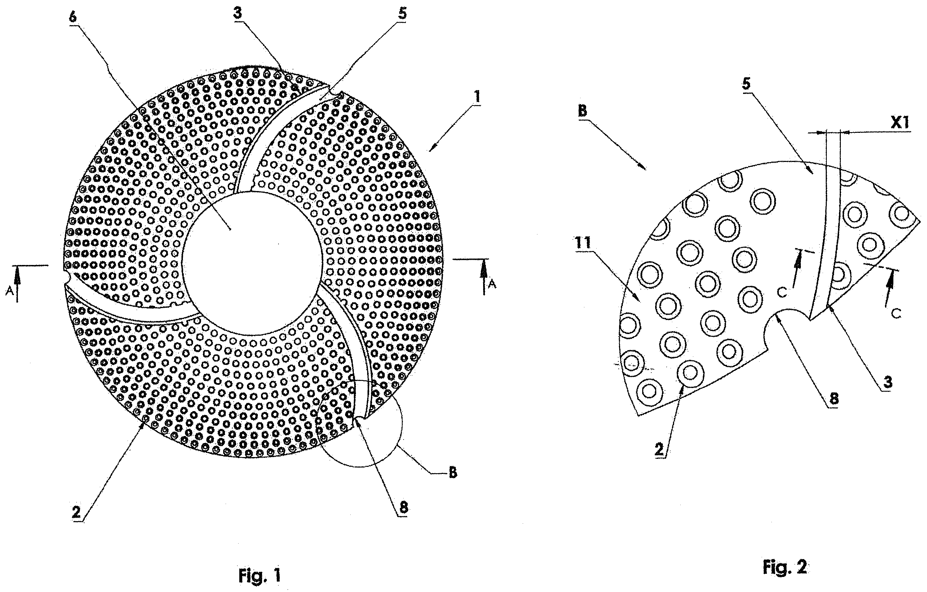

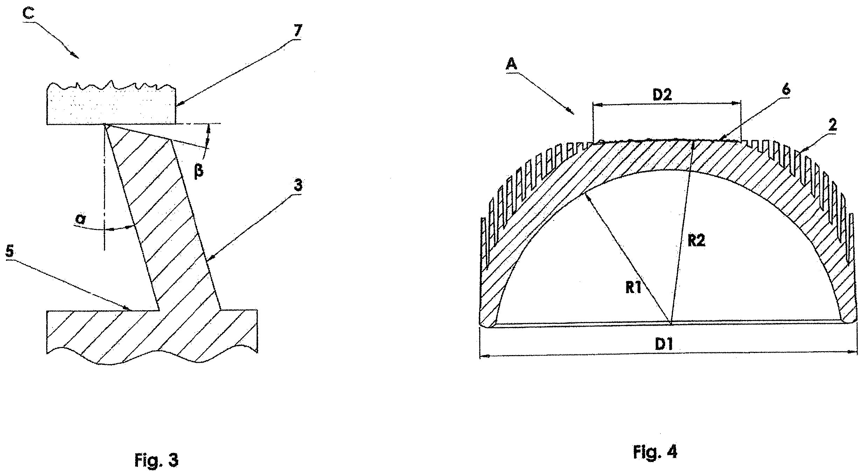

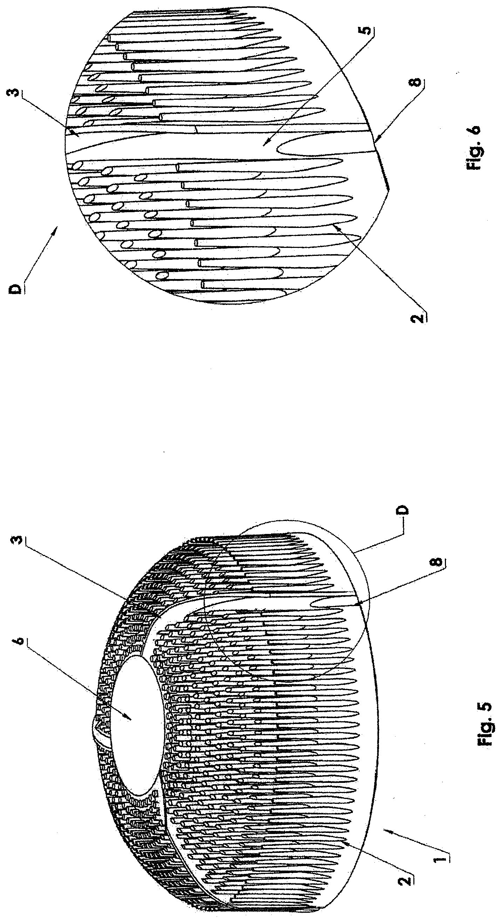

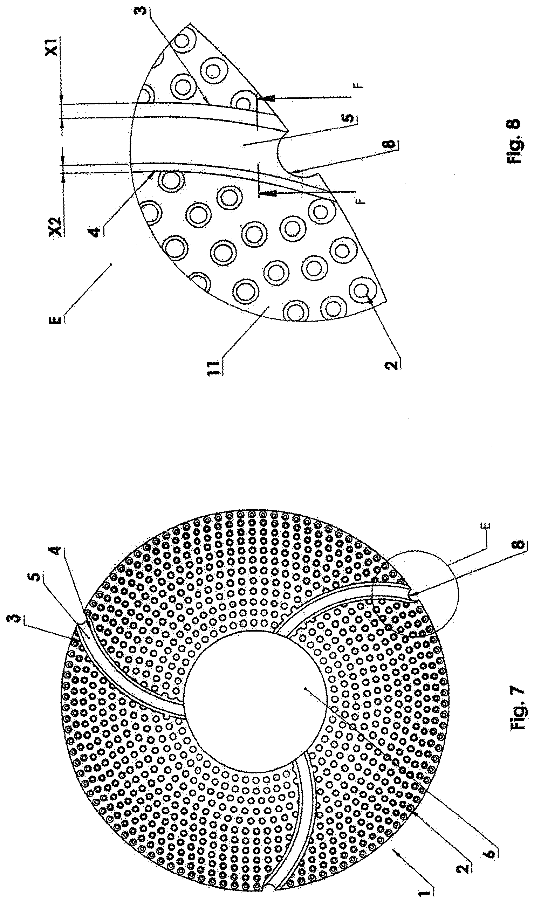

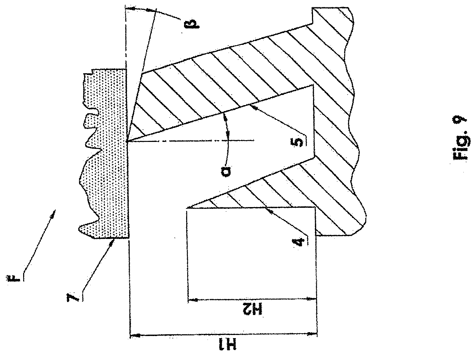

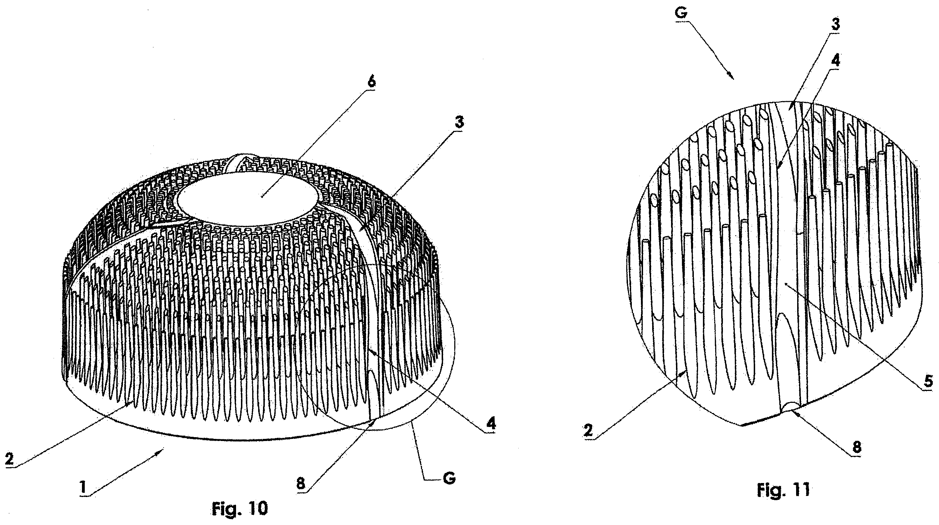

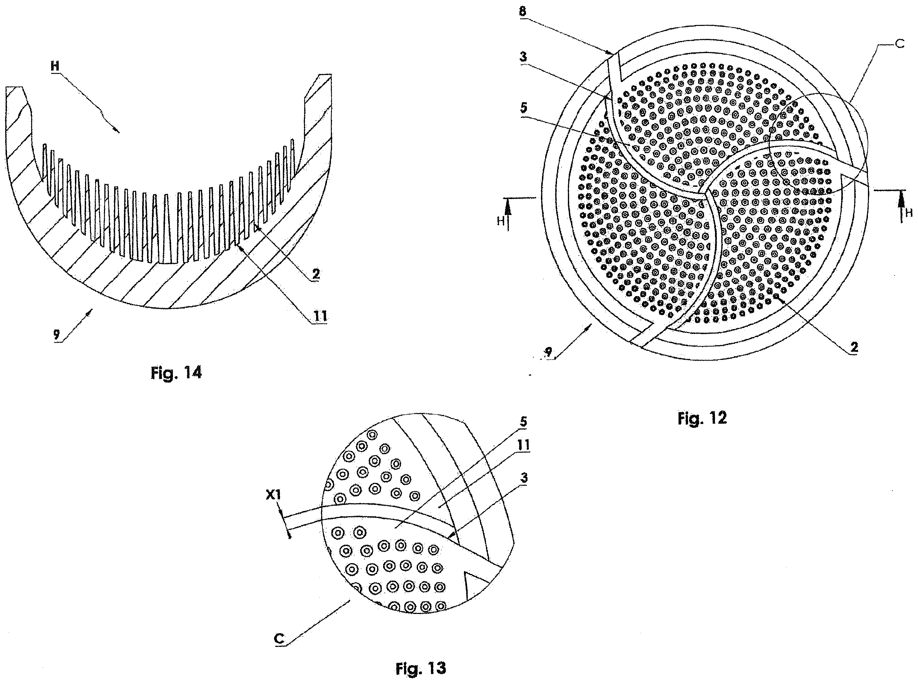

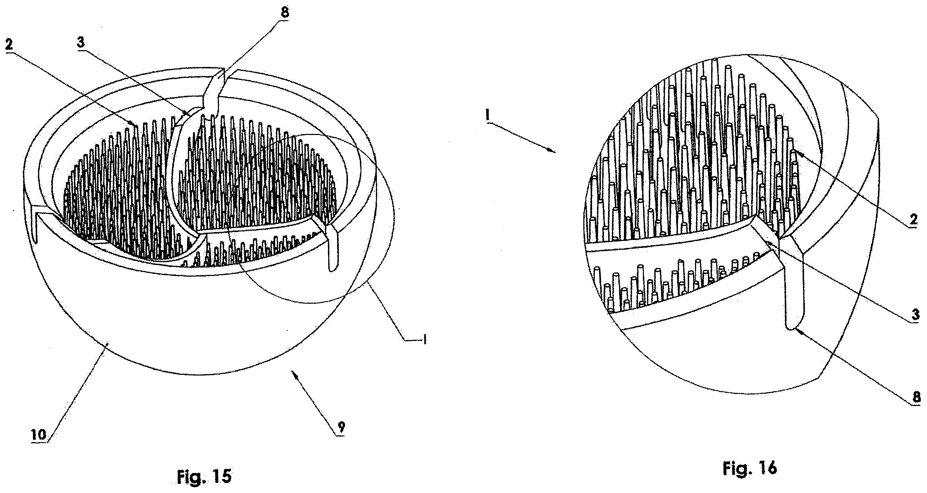

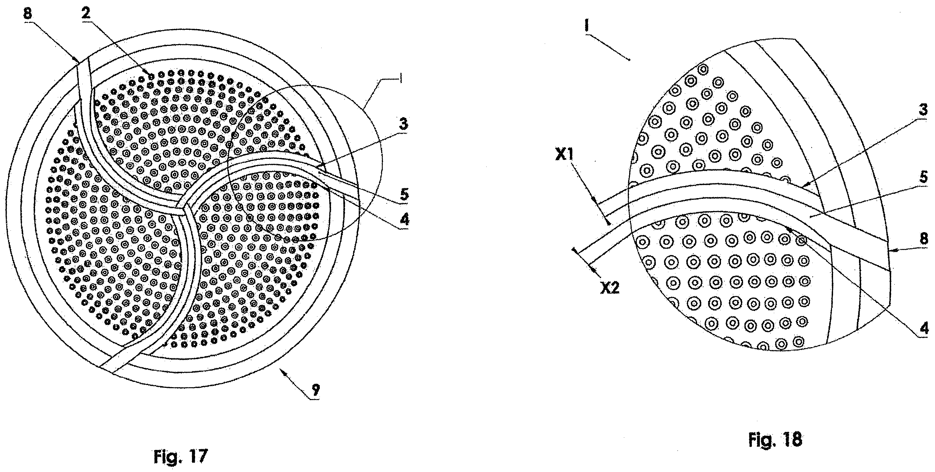

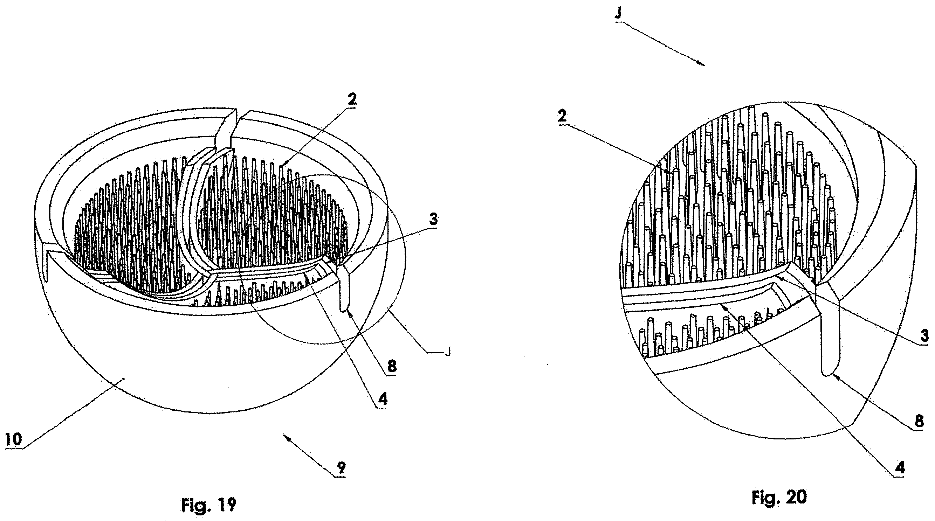

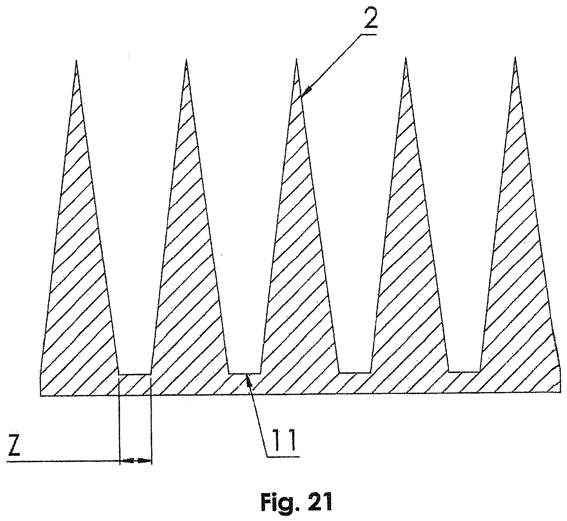

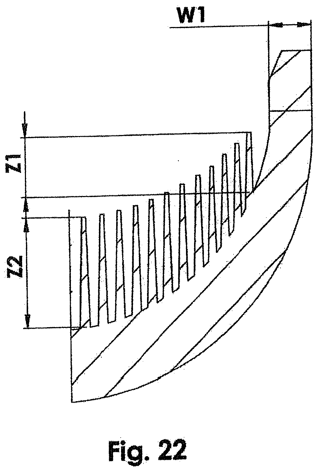

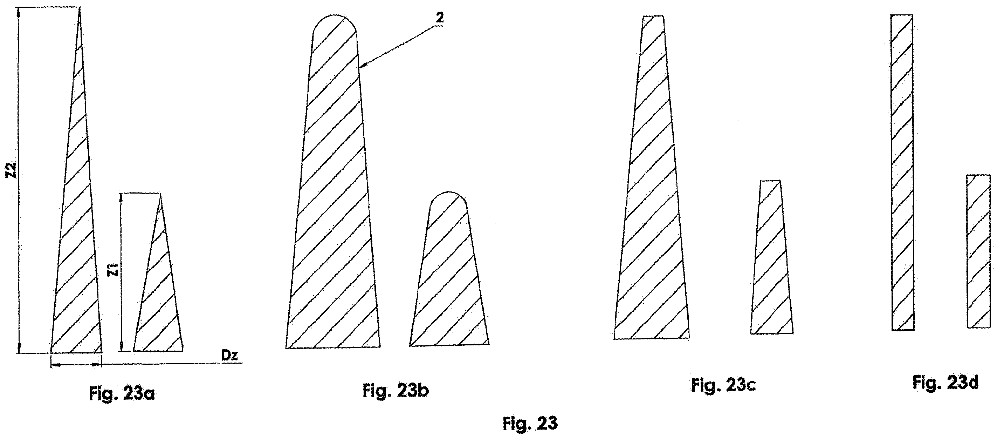

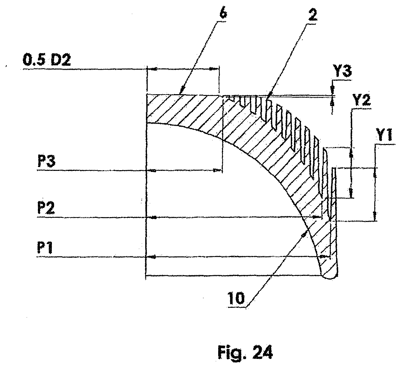

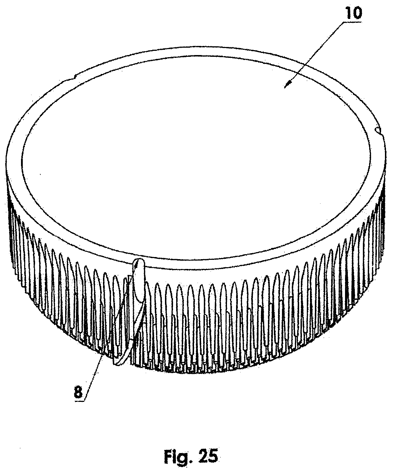

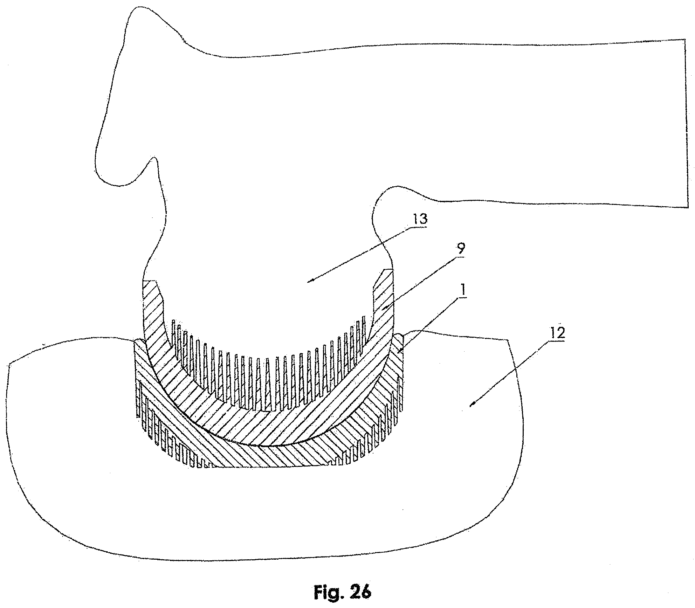

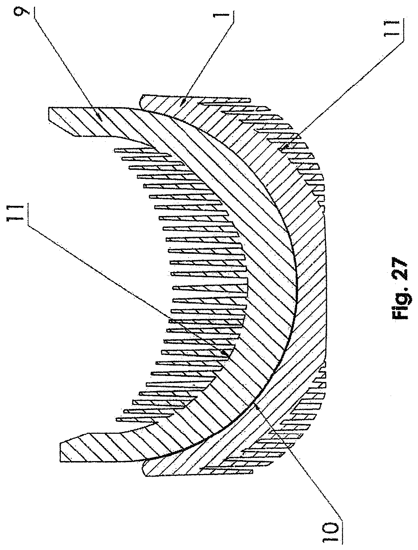

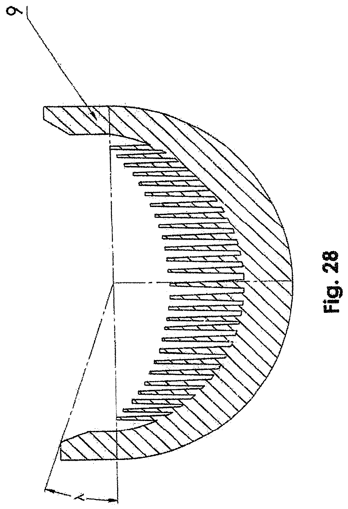

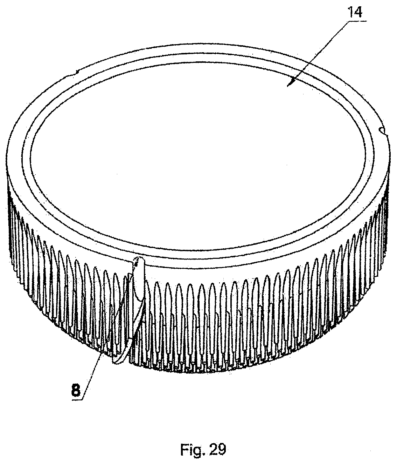

An example implementation of the invention is presented in the drawings, where fig. 1 presents a view of the acetabulum on the side of the supporting surface, fig. 2 presents a detail of fig. 1, fig. 3 presents a cross-section of a cutting blade, and fig. 4 a cross-section of the acetabulum, fig. 5 demonstrates a view of the acetabulum, fig. 6 presents a detail of the acetabulum from fig. 5, fig. 7 presents a view of the acetabulum on the side of the supporting surface, fig. 8 demonstrates a detail of the acetabulum from fig. 7, fig. 9 presents a cross-section of the cutting blade, fig. 10 presents a view of the acetabulum, fig. 11 presents a detail of the acetabulum from fig. 10, fig. 12 presents a view of the femoral head, fig. 13 presents a detail of the view from fig. 12, fig. 14 demonstrates a cross-section of the femoral head, fig. 15 presents a view of the femoral head on the side of the limiting surface, fig. 16 presents a detail of the view from fig. 15, fig. 17 presents a view of the femoral head, and fig. 18 a detail of the view of the femoral head from fig. 17, fig. 19 presents a view of the femoral head on the side of the limiting surface, fig. 20 a detail of the femoral head from fig. 19, fig. 21 presents a cross-section of the pins, and fig. 22 demonstrates a fragment of the cross-section of the femoral head, fig. 23 a cross-section of the pin, fig. 23a presents a pin in the form of a cone, fig. 23b a pin in the form of a rounded cone, fig. 23c a pin in the form of a prism, fig. 23d a pin in the form of a cuboid, fig. 24 presents a fragment of the cross-section of the acetabulum, fig. 25 presents an isometric view of the acetabulum on the side of contact of friction couple, fig. 26 presents a cross-section of the endoprosthesis acetabulum and femoral head system embedded on bone surfaces, fig. 27 presents a cross-section of the friction couple, and fig. 28 presents a cross-section of the femoral head endoprosthesis with a demonstrated angle of elongation of the cylindrical surface.

Hip joint endoprosthesis for implantation in bone surgery of ball-and-socket joints, in particular hip joint, consists of a moving connection provided by the contact surface 10 of two modules of the endoprosthesis femoral head 9 and acetabulum 1. The surfaces of the endoprosthesis femoral head 9 and acetabulum 1 which are in contact with the bone next to the joint have a shape similar to a sphere, on which pins 2 are placed. The pins 2 have an axis in principle parallel to the longitudinal axis of the endoprosthesis and at least two cutting blades 3 with an arc-shaped cross-section outline are formed, creating a groove 5 for removal of the products of cutting. In the endoprosthesis the sphere is a convex shell, on which pins 2 are located, forming the endoprosthesis acetabulum 1. The sphere is a concave shell, on which pins 2 are located, forming the endoprosthesis femoral head 9. The endoprosthesis has on its spherical surface, in the zone of the lengthwise axis, advantageously rounded, porous supporting surface 6. The pins 2 are placed in the area outside of the supporting surface 6, forming the limiting surface 11 and are placed on a spherical surface forming the limiting surface 11. In the endoprosthesis the cutting blades 3 have an angle of application g within a range of 5-15°, advantageously 10°, angle of attack α 35-45°, advantageously 40% and the wall thickness H of the cutting blade 3 wall amounting to 0.25-1 mm, advantageously 0.5 mm, and the H1 height of the cutting blade 3 is 2-3 times higher than the H2 height of the opposite wall 4 of the groove 5. The supporting surface 6 has a D2 diameter with a value of 1/2-1/3 of the D1 diameter of the femoral head 1. The femoral head endoprosthesis has a cylindrical elongation with an A angle in relation to the centre point within a range of 30 to 50 degrees. In the endoprosthesis the end of the grooves 5 is situated in the zone of technological gripping devices 8. The distance between the bases of adjacent pins 2 is at least 100 micrometers. The pins 2 have a shape of a cone or a rounded cone or a truncated cone or a regular polyhedron. Moreover the pins 2 have smooth side surfaces or shaped side surfaces. The height of pins 2 decreases from the edge to the supporting surface 6 and from the central axis of the endoprosthesis to the external direction. The height of the highest pin 2 is approximately twice the height of the lowest pin 2. The top points of the pins 2 create a surface with an outline similar to the surface of the sphere on which they are placed. The pins 2 are placed on an external spherical surface of the acetabulum 1 and on an internal spherical surface of femoral head 9. Additionally an insert 14 is introduced or not on the internal spherical surface of the endoprosthesis acetabulum 1.

The endoprosthesis consists of acetabulum 1 and femoral head 9, which on their limit surfaces in the form of spherical shells have polygonal needle protrusions in the form of pins 2 with parallel axes and varying length, the total area of which is a multiple of the joint area, whereas the minimum distance "z" between the bases of pins 2 is 100 micrometers, used in order to enable growth of trabeculae. The bases of pins 2 are not tangential. The axes of pins 2 are perpendicular to the plane of the supporting surface 6 of the femoral head and of the supporting surface 6 of the acetabulum 1, respectively. Additionally in order to reduce the abrasive wear of the friction couple of the acetabulum 1 and femoral head 9 of the hip joint endoprosthesis an insert 14 is introduced or not on the internal spherical surface of the endoprosthesis acetabulum 1 made of non-metallic material.

A precondition for the operation of the system is the fitting of the shape and size of the endoprosthesis femoral head 9 to the size and shape of the endoprosthesis acetabulum 1 in order to ensure the correct operation of the friction couple.

The endoprosthesis femoral head 9 is placed in the grips of an external device providing rotational movement, not shown on the drawing. This device moves the endoprosthesis around its angle of symmetry with a specific speed adapted to the machined material. The endoprosthesis, in the form of femoral head 9 is rotated, using the cutting blades 3 to remove bone tissue 7, which falls into the groove 5 between the wall of the cutting blade 3, and the wall 4 of the groove 5 or between the wall of the cutting blade 3 and the pins 2. The bone tissue 7 is moved by the pressure exerted by the newly cut bone tissue 7 between the wall of the cutting blade 3 and the wall 4 of the groove 5 or between the wall of the cutting blade 3 and the pins 2 out of the endoprosthesis, through the direct surface of the contact of the endoprosthesis with the bone tissue 7 surface. This process lasts until an optimum position of the endoprosthesis is obtained. The endoprosthesis causes a slight filling of the space between the pins 2 with small fragments of bone, which will feed the process of bone reconstruction and stabilization of the endoprosthesis. After removing the diseased tissue of the joint connection and using the cutting blades 3 to obtain a shape similar to the outline created by the tips of the pins 2, the endoprosthesis is disconnected from the device providing rotational movement, not shown of the drawing, and then is placed on the previously prepared surface and gradually inserted into the spongy bone space, in parallel to the axis of symmetry of a femoral head 13 of the joint. The supporting surface 6 does not contact the periosteum, the cortical substance and the spongy substance, into which the pins 2 at a distance longer than the plane of the supporting surface 6 are simultaneously inserted. The pins do not penetrate the spongy bone fully, to enable releasing of excessive intraosseous fluid pressure. The space between the pins 2 which is not filled with bone tissue 7 in this process will be filled with osteoblasts in the process of tissue regeneration, which will grow during the convalescence period.

The implantation method for acetabulum endoprosthesis is similar to the implantation of the femoral head, whereas the acetabulum endoprosthesis is implanted in such a manner that the supporting surface 6 of the truncated limiting surface adheres to the bone in a plane perpendicular to the axis of the central acetabulum 1 of the bone and be placed in a manner symmetric to this axis, and polygonal pins 2 in the area of the spherical belt on the limiting surface 11 of the endoprosthesis acetabulum 1 are partially sunk into the spongy structure of the bone. The remaining space between the polygonal needle protrusions 2 not sunk into the spongy bone, up to the limiting surface 11 are filled with osteoblasts which grow during the convalescence period as a result of lack of movement between the endoprosthesis acetabulum and the bone 12. The supporting surface 6 with a slight rounding protects the implanting process against possible trauma to blood vessels present in the direct vicinity of the cut tissue.

Additionally in order to reduce the abrasive wear of the friction couple of the acetabulum 1 and femoral head 9 of the hip joint endoprosthesis an insert 14 is introduced or not on the internal spherical surface of the endoprosthesis acetabulum 1 made of non-metallic material.

A precondition for the operation of the system is the fitting of the shape and size of the endoprosthesis femoral head 9 to the size and shape of the endoprosthesis acetabulum 1 in order to ensure the correct operation of the friction couple.

The pins 2 are partially sunk in the bone tissue 7, advantageously to half their height, in order to allow osteoblasts to grow on the surface which is not sunk in the bone tissue 7.

### Designation

1 - acetabulum
2 - pin
3 - cutting blade
4 - wall
5 - groove
6 - supporting surface
7 - bone tissue
8 - technological gripping device
9 - femoral head
10 - friction couple contact surface
11 - limiting surface
12 - pelvic bone
13 - femoral head of the joint
14 - insert
H - cutting edge 3 wall thickness
H1 - cutting edge 3 height
H2 - opposite wall 4 height
α - angle of attack angle of application
ß - angle of application
λ - angle of elongation of the femoral head 9 endoprosthesis body
D1- diameter of the femoral head 9
D2 - diameter of the supporting surface 6
z - distance between pins 2

## Claims

1. An endoprosthesis for implantation in bone surgery of ball-and-socket joints, in particular hip joint, the endoprosthesis consisting of a moving connection provided by a contact surface (10) of each of an endoprosthesis head component (9) and an endoprosthesis acetabular component (1), whereas each of the endoprosthesis head component (9) and the endoprosthesis acetabular component (1) has a bone contacting surface having a substantially spherical shape, on which a plurality of pins (2) are located, each of the pins (2) having an axis principally parallel to a lengthwise axis of the endoprosthesis, **characterized in that** the bone contacting surface of each of the endoprosthesis head component (9) and the endoprosthesis acetabular component (1) has at least two cutting blades (3) with each of the blades (3) having an arc-shaped cross-section outline,
wherein each of the blades (3) creates a groove (5) for removal of products of cutting.

2. An endoprosthesis in accordance with claim 1, wherein the endoprosthesis acetabular component (1) is a convex shell, on which the pins (2) are located externally.

3. An endoprosthesis in accordance with claim 1, wherein the endoprosthesis head component (9) is a concave shell, on which the pins (2) are located internally.

4. An endoprosthesis in accordance with claim 1 and 2, wherein each bone contacting surface has on its spherical surface, in a zone of the lengthwise axis, a rounded and porous supporting surface (6).

5. An endoprosthesis in accordance with claim 4, wherein the pins (2) are placed on the spherical surface in an area outside the supporting surface (6) forming a limiting surface (11).

6. An endoprosthesis in accordance with claim 1, wherein the cutting blades (3) have an angle of application (ß) within a range of 5-15°, advantageously 10°, an angle of attack (α) of 35-45°, advantageously 40°, and a wall thickness (H) of the cutting blade (3) of 0.25-1 mm, advantageously 0.5 mm, and a height (H1) H1 of the cutting blade 3 is 2-3 times higher than a height ( H2) of an opposite wall (4) of the groove (5).

7. An endoprosthesis in accordance with claim 1, wherein the supporting surface (6) has a diameter (D2) with a value of 1/2-1/3 of a diameter (D1) of the endoprosthesis head component (9).

8. An endoprosthesis in accordance with claim 1, wherein the endoprosthesis head component (9) has a cylindrical elongation defining an angle (λ) in relation to a centre point within a range of 30 to 50 degrees.

9. An endoprosthesis in accordance with claim 1, wherein an end of each groove (5) is situated in a zone of technological gripping devices (8).

10. An endoprosthesis in accordance with claim 1, wherein a distance (z) between bases of adjacent pins (2) is at least 100 micrometers.

11. An endoprosthesis in accordance with claim 1, wherein the pins (2) have a shape of a cone.

12. An endoprosthesis in accordance with claim 1, wherein the pins (2) have shaped side surfaces.

13. An endoprosthesis in accordance with claim 4, wherein a height of the pins (2) on the bone contacting surface of the endoprosthesis acetabular component (1) from an edge to the supporting surface (6).

14. An endoprosthesis in accordance with claim 1, wherein a height of the pins (2) on the bone contacting surface of the endoprosthesis head component decreases from the lengthwise axis of the endoprosthesis in an external direction.

15. An endoprosthesis in accordance with claim 1, comprising an optional insert (14) configured to be introduced on an internal spherical surface of the endoprosthesis acetabular component (1).

## Patentansprüche

1. Endoprothese zur Implantation im Rahmen der Chirurgie der Konchen und Gelenke, insbesondere des Hüftgelenks, wobei sich die Endoprothese aus einer, durch die Berührungsoberfläche (10) jedes der Elementen des Endoprothesen-Hüftkopfes (9) und der - Hüftgelenkspfanne (1) gebildeten beweglichen Verbindung zusammensetzt, wobei jedes der Elementen des Endoprothesen-Hüftkopfes (9) und der -Hüftgelenkspfanne (1) grundlegend eine kugelrunde Berührungsfläche mit dem Knochen hat, auf welcher sich mehrere Stifte (2) befinden, wobei jeder der Stifte (2) grundlegend eine zur Längsachse der Endoprothese parallele Achse hat, **charakterisiert dadurch, dass** die Berührungsfläche mit dem Konchen jedes der Elemente des Endoprothese-Hüftkopfes (9) und der -Hüftgelenkspfanne (1) mindestens zwei Schneidklingen (3) hat, wo jede Schneidklinge (3) einen bogenförmigen Querschnittumriss hat, wobei jede Schneidklinge (3) einen Schlitz (5) zur Entfernung von Produkten des Schneidens bildet.

2. Endoprothese gemäß dem Anspruch 1, **dadurch gekennzeichnet, dass** das Hüftgelenkspfanne-Element (1) der Endoprothese ein konvexer Überzug ist, auf welchem Stifte (2) auf der Außenseite angebracht sind.

3. Endoprothese gemäß dem Anspruch 1, **dadurch gekennzeichnet, dass** das Hüftkopf-Element (9) der Endoprothese ein konkaver Überzug ist, auf welchem Stifte (2) auf der Innenseite angebracht sind.

4. Endoprothese gemäß den Ansprüchen 1 und 2, **dadurch gekennzeichnet, dass** jede mit dem Knochen in Berührung kommende Fläche auf ihrer kugelrunden Oberfläche, im Längsachsenbereich, eine abgerundete und poröse Stützfläche (6) hat.

5. Endoprothese gemäß dem Anspruch 4, **dadurch gekennzeichnet, dass** die Stifte (2) auf einer kugelrunden Fläche im Bereich außerhalb der Stützfläche (6) angebracht sind und eine umgrenzende Fläche (11) bilden.

6. Endoprothese gemäß dem Anspruch 1, **dadurch gekennzeichnet, dass** die Schneidklingen (3) einen Freiwinkel β im Bereich 5° - 15°, vorteilhaft 10°, den Spanwinkel α im Bereich 35° - 45°, vorteilhaft 40° und eine Wanddicke H der Schneidklinge (3) von 0,25 - 1 mm, vorteilhaft 0,5 mm aufweist, und die Höhe H1 der Schneidklinge (3) 2- bis 3-mal größer als die Höhe H2 der gegenüberliegenden Wand (4) des Schlitzes (5) ist.

7. Endoprothese gemäß dem Anspruch 1, **dadurch gekennzeichnet, dass** die Stützfläche (6) einen Durchmesser D2 mit einem Wert von 1/2 - 1/3 des Durchmessers D1 vom Hüftkopf-Element (9) der Endoprothese hat.

8. Endoprothese gemäß dem Anspruch 1, **dadurch gekennzeichnet, dass** das Hüftkopf-Element (9) der Endoprothese eine zylindrische Verlängerung hat, die den Winkel λ zum Mittelpunkt im Bereich von 30° bis 50° definiert.

9. Endoprothese gemäß dem Anspruch 1, **dadurch gekennzeichnet, dass** das Ende jedes Schlitzes (5) im Arbeitsbereich der technologischen Fangvorrichtungen (8) positioniert ist.

10. Endoprothese gemäß dem Anspruch 1, **dadurch gekennzeichnet, dass** der Abstand (z) zwischen den Füßen von benachbarten Stiften (2) mindestens 100 Mikrometer beträgt.

11. Endoprothese gemäß dem Anspruch 1, **dadurch gekennzeichnet, dass** die Stifte (2) kegelförmig sind.

12. Endoprothese gemäß dem Anspruch 1, **dadurch gekennzeichnet, dass** die Stifte (2) gestaltete Seitenflächen aufweisen.

13. Endoprothese gemäß dem Anspruch 4, **dadurch gekennzeichnet, dass** die Höhe der Stifte (2) auf der Fläche, wo das Hüftgelenkspfanne-Element (1) der Endoprothese mit dem Knochen in Berührung kommt, im Bereich von der Kante bis zur Stützfläche (6) immer kleiner wird.

14. Endoprothese gemäß dem Anspruch 1, **dadurch gekennzeichnet, dass** die Höhe der Stifte (2) auf der Fläche, wo das Gelenkkopf-Element (9) der Endoprothese mit dem Knochen in Berührung kommt, in der Richtung von der Endoprothese-Längsachse nach Außen immer kleiner wird.

15. Endoprothese gemäß dem Anspruch 1, **dadurch gekennzeichnet, dass** sie eine optionale, zum Einbringen auf die innere kugelförmige Fläche des Hüftgelenkspfanne-Elements (1) der Endoprothese konfigurierte Einlage (14) beinhaltet.

## Revendications

1. Endoprothèse pour l'implantation dans la chirurgie osseuse des articulations sphériques, en particulier l'articulation de la hanche, l'endoprothèse consistant en une connexion mobile fournie par une surface de contact (10) de chacun d'un composant de tête d'endoprothèse (9) et d'un élément acétabulaire d'endoprothèse - acétabulum ou cotyle (1), alors que chacun du composant de tête d'endoprothèse (9) et de l'élément acétabulaire d'endoprothèse - acétabulum ou cotyle (1) a une surface de contact avec l'os ayant une forme substantiellement sphérique, sur laquelle se trouvent plusieurs broches (2), chacune des broches (2) ayant un axe principalement parallèle à un axe longitudinal de l'endoprothèse, **caractérisée en ce que** la surface de contact avec l'os du composant de tête d'endoprothèse (9) et de l'élément acétabulaire d'endoprothèse- acétabulum ou cotyle (1) comporte au moins deux lames de coupe (3), chacune des lames (3) ayant un contour de section transversale en forme d'arc, dans laquelle chacune des lames (3) crée une rainure (5) pour l'élimination des produits de la coupe.

2. Endoprothèse selon la revendication 1, dans laquelle de l'élément acétabulaire de l'endoprothèse - acétabulum ou cotyle (1) est une coque convexe, sur laquelle les broches (2) sont situées à l'extérieur.

3. Endoprothèse selon la revendication 1, dans laquelle le composant de la tête de l'endoprothèse (9) est une coquille concave, sur laquelle les broches (2) sont situées à l'intérieur.

4. Endoprothèse selon les revendications 1 et 2, dans laquelle chaque surface de contact avec l'os présente sur sa surface sphérique, dans une zone de l'axe longitudinal, une surface d'appui arrondie et poreuse (6).

5. Endoprothèse selon la revendication 4, dans laquelle les broches (2) sont placées sur la surface sphérique dans une zone à l'extérieur de la surface de support (6) formant une surface de limitation (11).

6. Endoprothèse selon la revendication 1, dans laquelle les lames de coupe (3) ont un angle d'application ß compris entre 5 et 15°, avantageusement 10°, un angle d'attaque α de 35-45°, avantageusement 40°, et une épaisseur de paroi H de la lame de coupe (3) de 0,25-1 mm, avantageusement 0,5 mm, et une hauteur H1 de la lame de coupe (3) est 2 à 3 fois plus élevée qu'une hauteur H2 d'une paroi opposée (4) de la rainure (5).

7. Endoprothèse selon la revendication 1, dans laquelle la surface d'appui (6) a un diamètre D2 d'une valeur de 1/2-1/3 du diamètre D1 du composant de la tête de l'endoprothèse (9).

8. Endoprothèse selon la revendication 1, dans laquelle le composant de tête d'endoprothèse (9) présente une élongation cylindrique définissant un angle λ par rapport à un point central dans une plage de 30 à 50 degrés.

9. Endoprothèse selon la revendication 1, dans laquelle une extrémité de chaque rainure (5) est située dans une zone de dispositifs de préhension technologiques (8).

10. Endoprothèse selon la revendication 1, dans laquelle une distance (z) entre les bases des broches adjacentes (2) est d'au moins 100 micromètres.

11. Endoprothèse selon la revendication 1, dans laquelle les broches (2) ont une forme de cône.

12. Endoprothèse selon la revendication 1, dans laquelle les broches (2) ont des surfaces latérales façonnées.

13. Endoprothèse selon la revendication 4, dans laquelle la hauteur des broches (2) sur la surface en contact avec l'os de l'élément acétabulaire de l'endoprothèse - acétabulum ou cotyle (1) diminue d'un bord à la surface d'appui (6).

14. Endoprothèse selon la revendication 1, dans laquelle la hauteur des broches (2) sur la surface de contact avec l'os du composant de la tête de l'endoprothèse (9) diminue par rapport à l'axe longitudinal de l'endoprothèse dans une direction externe.

15. Endoprothèse selon la revendication 1, comprenant un insert optionnel (14) configuré pour être introduit sur une surface sphérique interne de l'élément acétabulaire de l'endoprothèse - acétabulum ou cotyle (1).
